Europäisches Patentamt

European Patent Office

Office européen des brevets

(11) Publication number: **0 358 325**
A1

(12) **EUROPEAN PATENT APPLICATION**

(21) Application number: 89307853.5

(22) Date of filing: 02.08.89

(51) Int. Cl.⁵: **C12N 15/81 , C12N 15/54 , C12N 9/12 , C12N 1/18 , //(C12N1/18,C12R1:865)**

The applicant has filed a statement in accordance with Rule 28 (4) EPC (issue of a sample only to an expert). Accession number(s) of the deposit(s): FERM BP-1948, BP-1976, BP-1977, BP-2090, with Fermentation Research Institute. Japan

(30) Priority: 10.08.88 JP 197965/88

(43) Date of publication of application:
**14.03.90 Bulletin 90/11**

(84) Designated Contracting States:
**AT BE CH DE ES FR GB GR IT LI LU NL SE**

(71) Applicant: **TAKEDA CHEMICAL INDUSTRIES, LTD.**
3-6, Doshomachi 2-chome Chuo-ku
Osaka 541(JP)

(72) Inventor: **Ono, Hideo**
12-7, Uzumoridai 4-chome
**Higashinada-ku Kobe Hyogo 658(JP)**
Inventor: **Fukusumi, Shoji**
50-1, Yamadaminami
**Suita Osaka 565(JP)**
Inventor: **Fujisama, Yukio**
31-104, 1 Mikagenakamachi 4-chome
**Higashinada-ku**
**Kobe Hyogo 658(JP)**

(74) Representative: **Laredo, Jack Joseph et al**
**Elkington and Fife Beacon House 113**
**Kingsway**
**London, WC2B 6PP(GB)**

(54) **Production of protein kinase C by yeast.**

(57) Disclosed are an expression vector for a protein kinase C protein, a yeast transformed with said vector, and a method of producing the protein by means of said yeast transformant. This process provides for effective large-scale production of protein kinase C protein with enzymatic activity, which can be used as a biochemical reagent, or as a diagnostic or analytical reagent.

EP 0 358 325 A1

Xerox Copy Centre

## PRODUCTION OF PROTEIN KINASE C BY YEAST

### BACKGROUND OF THE INVENTION

This invention relates to a process which employs yeast to produce a protein kinase C useful as a biochemical and/or diagnostic reagent. Further, this invention relates to a protein kinase C expression vector and to yeast transformed thereby.

Numerous kinds of biologically active substances such as cell growth factors, hormones and neurotransmitters are known to participate in the exertion of various functions and adaptation phenomena of living organisms. These extracellular signals are exerted through intracellular mediators such as cyclic AMP, cyclic GMP, diacylglycerol and calcium. In particular, it has been revealed that diacylglycerol is produced as a result of the decomposition of inositol phospholipids in cell membranes by a number of hormones and neurotransmitters which are responsible for the activation of cellular functions. It is usually considered that diacylglycerol activates a protein kinase C in the presence of calcium and that the phosphorylation of various intercellular proteins with this enzyme causes the activation of various cellular functions and cellular proliferation. Thus, the protein kinase C is an enzyme which plays an important role in one of the main information transmission mechanisms of external signals. The enzyme isolated from rat brains is an acidic protein having an isoelectric point of pH 5.6 and a molecular weight of approximately 77,000. This enzyme is composed of a single peptide having a hydrophobic region serving as a binding site to cell membranes and a hydrophilic region in which an active center exists. The protein kinase C is normally in an inactive form, but is activated by calcium, phosphatidylserine and diacylglycerol. ATP is known to serve as a phosphoric acid donor.

Thus, the protein kinase C, as one of the protein phosphorylation enzymes, also transmits the external signals into cells through the phosphorylation of the proteins. This is therefore an indispensable enzyme in studying extracellular signal reception and transmission mechanisms, and is very valuable as a reagent. This enzyme is also activated directly by phorbol esters which act as tumor promoters. The phorbol esters are known not only to cause promotion of carcinogenesis, but also to take part in various biological reactions such as mitosis and differentiation of cells, induction of enzymes and acceleration of lipid metabolism. In these circumstances, there is a great possibility that the protein kinase C becomes an important index enzyme in diseased cells or tumor cells resulting from disturbances of cellular signal reception and transduction mechanisms, and antibodies to the protein kinase C have been proposed for use as diagnostic or analytical reagents. For these purposes, it is essential to provide a sufficient amount of protein kinase C.

However, it is very difficult to obtain large quantities of protein kinase C even when the enzyme can be extracted from relatively easily available animal materials. A rat protein kinase C has already been purified from rat brains. In this case, some subtypes have already been successfully cloned [see Y. Ono et al., Science 236, 1116 (1987)], and their proteins have also been produced by gene recombinant techniques. However, the proteins are produced in small amounts due to the limitations of gene expression inherent in animal cells. It has been therefore desired to provide a method for obtaining the protein kinase C in a larger amount.

Furthermore, many of the properties of a human protein kinase C and the rat protein kinase C are unknown. It has been therefore desired to provide a method for producing the protein as a reagent or an analytical agent by gene recombinant techniques in a large amount.

### SUMMARY OF THE INVENTION

The present invention provides:

(1) a protein kinase expression vector which contains a DNA sequence coding for a protein kinase C protein and of which autonomous replication in yeast is possible;

(2) yeast transformed by using the expression vector described in the above item (1);

(3) yeast described in the above item (1), wherein the yeast is Saccharomyces cerevisiae;

(4) yeast described in the above item (3), wherein the yeast is a respiratory deficient strain of Saccharomyces cerevisiae; and

(5) a process for producing a protein which comprises cultivating the yeast described in the above item (2) in a culture medium, and forming and accumulating a protein kinase C protein in a cell.

BRIEF DESCRIPTION OF THE DRAWINGS

Fig. 1 is a schematic representation illustrating a process for preparing expression vector pTFE112;

Fig. 2 shows the DNA sequence of an improved hen egg white lysozyme signal peptide;

Fig. 3 shows the DNA sequence of a multi-linker;

Fig. 4 shows the DNA sequence of a signal peptide alternative to the peptide shown in Fig. 2;

Fig. 5 is a schematic representation illustrating the construction of a 2.0 kbp fragment of Pstl-Sacl coding for a PKCα gene;

Fig. 6 is a schematic representation illustrating the construction of plasmid pTFE755; and

Fig. 7 is a schematic representation illustrating the construction of plasmid pTFE756.

DESCRIPTION OF THE PREFERRED EMBODIMENTS

In this invention, any vector (for example, a plasmid) may be used as long as it is capable of replicating in yeast. Examples of the vectors include pSH19 [S. Harashima et al., Mol. Cell. Biol. 4, 771(1984)] and pSH19-1 (European Patent Publication No. EP-A-0235430). A vehicle for expressing a foreign gene is obtained by inserting a promoter in the vector.

Any promoter for gene expression can be used as long as it functions in the yeast. For example, there are preferably used a GLD (GAPH: glyceraldehyde-3-phosphate dehydrogenase) promoter, a PHO(acid phosphatase)5 promoter, a PGK(phosphoglycerate kinase) promoter, an ADH(alcohol dehydrogenase) promoter, a PHO81 promoter and a GAL(UDP-glucose 4-epimerase)10 promoter. The promoters can be prepared from the corresponding genes by enzymatic techniques, and can also be chemically synthesized.

The plasmid for expressing a DNA sequence coding for a protein kinase C can be obtained by inserting the DNA sequence coding for the protein kinase C, or a DNA sequence in which a DNA sequence coding for all or a part of a signal peptide functioning in the yeast is ligated to the 5'-terminus of the DNA sequence coding for the protein kinase C, into the expression vector described above at a position downstream from the promoter. Examples of the expression vectors include pTFE112, pPHO17 and pGLD906-1 [Y. Itoh et al., Biochem. Biophys. Res. Commun. 138, 268 (1986)]. In this case, the DNA expression can be enhanced by inserting a terminator into the vector at a position downstream from the DNA sequence coding for the protein kinase C. Examples of the terminators include a PGK gene, a FLP-(site-specific recombination) gene of 2 μ DNA and an invertase gene (SUC2).

The polypeptide kinase C in the present invention includes one derived from human, bovine, rat or rabbit. For example, there are the protein kinases C described in Table 1 on page 1117 of Science 236, 1116 - 1120 (1987).

The expression vector containing the DNA sequence having a nucleotide sequence coding for the polypeptide of the protein kinase C according to the present invention is produced, for example, by the following steps:

(a) separating an RNA coding for a protein kinase C,

(b) preparing a single-stranded complementary DNA (cDNA) from the RNA, and then a double-stranded DNA therefrom,

(c) inserting the complementary DNA into a plasmid,

(d) transforming a host with the resulting recombinant plasmid,

(e) cultivating the transformant thus obtained, and then isolating a plasmid containing a desired DNA from the transformant by any suitable method such as a colony hybridization method using a rat cDNA as a probe,

(f) cutting out the desired, cloned DNA from the plasmid, and

·(g) ligating the cloned DNA to a vehicle at a position downstream from a promoter.

The RNA coding for the protein kinase C may be obtained from various kinase C-producing cell lines such as brain-derived cells and fibroblasts. Examples of human fibroblasts include WI38 (ATCC No. CCL-75) and IMR90 (ATCC No. CCL-186). The above-mentioned cells WI38 and IMR90 are listed in Catalogue of Cell Lines & Hybridomas, 5th edition, 1985, published by The American Type Culture Collection.

Methods for preparing the RNAs from the kinase C-producing cell lines include the guanidine-thiocyanate method [J. M. Chirgwin et al., Biochemistry 18, 5294 (1979)].

Using the RNA thus obtained as a template, the cDNA is synthesized with a reverse transcription enzyme, for example, according to the method of H. Okayama et al. [Molecular and Cellular Biology 2, 161 (1982); ibid. 3, 280 (1983)]. The resulting cDNA is inserted into the plasmid.

Methods for constructing the expression plasmids in the present invention are known in the art and described in literatures such as Molecular Cloning (1982) published by Cold Spring Harbor Laboratory.

3

The hosts transformed with the expression plasmid include yeast, preferably Saccharomyces cerevisiae. In particular, respiratory-deficient strains ($\rho^-$) of Saccharomyces cerevisiae are suitable as hosts.

Methods for obtaining respiratory-deficient strains ($\rho^-$) from the yeast parent strain ($\rho^+$) having a respiratory activity are known per se and described, for example, in Laboratory Course Manual for Methods in Yeast Genetics (1986) published by Cold Spring Harbor Laboratory. That is, respiratory-deficient strains can be easily obtained by cultivating the parent strain in a medium containing ethidium bromide and then isolating strains which can grow in a medium containing glucose as a carbon source, but can not grow in a medium containing glycerol. Although the frequency is low, respiratory-deficient strains can be obtained by single colony isolation from the parent strain. When the parent strain is the transformant (recombinant) having the expression plasmid, the desired recombinant having a high gene expression amount can be directly obtained by isolating its respiratory-deficient strain. When the parent strain carries no expression plasmid, the desired recombinant can be obtained by isolating its respiratory-deficient strain and then introducing an expression plasmid therein.

Methods for transforming the yeast by using the recombinant DNA are known per se and include, for example, the lithium method [H. Itoh et al., J. Bacteriol. 153, 163 (1983) and the protoplast method [A. Hinnen et al., Proc. Natl. Acad. Sci. U.S.A. 75, 1927(1978)].

The transformant (recombinant) thus obtained is cultivated by methods which are known per se.

Examples of the media include Burkholder minimum medium [K. L. Bostian et al., Amer. J. Bot. 30, 206 (1943)], its modified medium [A. Toh-e et al., J. Bacteriol. 113, 727 (1973)] and lower phosphate medium [A. Toh-e et al., J. Bacteriol. 113, 727 (1973)]. The cultivation is generally conducted at 15 to 40°C, preferably 24 to 37°C for 10 to 168 hours, preferably 72 to 144 hours with or without shaking, with aeration or agitation if necessary.

The protein kinase C thus formed and accumulated can be easily purified by known protein extraction and purification methods, for example, suitable combinations of purification procedures such as cellular crushing with glass beads, centrifugation, salt precipitation, electrofocusing, gel filtration, ion exchange chromatography, high performance liquid chromatography, affinity chromatography, sucrose density-gradient ultracentrifugation and cesium chloride density-gradient ultracentrifugation.

The protein kinase C polypeptide obtained according to the present invention has enzyme activity similar to that of the protein kinase C polypeptide prepared from rat brain cells and hence can be used as a reagent or a diagnostic kit.

The protein kinase C thus produced can be used as a reagent, or as a diagnostic or analytical agent in conjunction with an antibody produced therefrom. For example, the protein kinase C obtained according to the present invention can be used as a reagent for investigating cellular signal transmission mechanisms, as a reagent for diagnosis or analysis of diseases (such as tumor) resulting from a disturbance of the cellular signal transmission mechanisms, or as an agent for screening inhibitors or therapeutic agents to the diseases. The protein kinase C is used, for example, in an amount of about 0.001 to $\mu$g per diagnostic or screening test.

According to the present invention, a large amount of the protein kinase C can be expressed in the yeast. Therefore, the large-scale, effective production of the protein kinase C is possible.

In the present specification and the accompanying drawings, the abbreviations of bases, amino acids and the like are based on those adopted by IUPAC-IUB Committee on Biochemical Nomenclature or those customarily used in the art. Examples of the abbreviations are as follows:

DNA : Deoxyribonucleic acid
RNA : Ribonucleic acid
mRNA : Messenger ribonucleic acid
A : Adenine
T : Thymine
G : Guanine
C : Cytosine
dATP : Deoxyadenosine triphosphate
dTTP : Deoxythymidine triphosphate
dGTP : Deoxyguanosine triphosphate
dCTP : Deoxycytidine triphosphate
ATP : Adenosine triphosphate
EDTA : Ethylenediaminetetraacetic acid
SDS : Sodium dodecylsulfate
DTT : Dithiothreitol
Gly : Glycine (G)

EP 0 358 325 A1

Ala : Alanine (A)
Val : Valine (V)
Leu : Leucine (L) Ile : Isoleucine (I)
Ser : Serine (S)
Thr : Threonine (T)
Cys : Cysteine (C)
1/2Cys : Half cystine
Met : Methionine (M)
Glu : Glutamic acid (E)
Asp : Aspartic acid (D)
Lys : Lysine (K)
Arg : Arginine (R)
His : Histidine (H)
Phe : Phenylalanine (F)
Tyr : Tyrosine (Y)
Trp : Tryptophan (W)
Pro : Proline (P)
Asn : Asparagine (N)
Gln : Glutamine (Q)
$Ap^r$ : Ampicillin-resistant gene
$Tc^r$ : Tetracycline-resistant gene
ARS 1 : Autonomous replication sequence 1

When the optical isomers are capable of existing with respect to the amino acids, the L-amino acids are represented unless otherwise specified.

As used herein, the term "protein Kinase C protein" means a polypeptide or protein which is dependent to phospholipid or calcium ion, is activated by diacylglycerol or phorbol ester, and has an activity of phosphorylation of serine or threonine residue of a protein.

The present invention will hereinafter be described in detail with the following Reference Examples and Examples. It is understood of course that these Reference Examples and Examples are not intended to limit the scope of the inventions.

The respiratory-deficient strain Saccharomyces cerevisiae NA74-3A ($\rho^-$) obtained idn Reference Example 1 has been deposited in the Institute for Fermentation, Osaka, Japan (hereinafter referred to as IFO) with the accession number IFO 10431 since October 23, 1987. This transformant was deposited in Fermentation Research Institute, Agency of Industrial Science and Technology, Ministry of International Trade and Industry, Japan (hereinafter referred to as FRI) with the accession number FERM P-9692 on November 5, 1987. This deposit has been converted to the deposit under the Budapest Treaty and the transformant has been stored at FRI with the accession number FERM BP-1948.

The transformant Saccharomyces cerevisiae NA74-3A/pTFL710T carrying the plasmid pTFL710T obtained in Reference Example 5 has been deposited in IFO with the accession number IFO 10432 since October 23, 1987 and in FRI with the accession number FERM P-9693 since November 5, 1987. This deposit has been converted to the deposit under the Budapest Treaty and the transformant has been stored at FRI with the accession number FERM BP-2090.

The transformants Saccharomyces cerevisiae NA74-3A ($\rho^-$)/pTFE755 and Saccharomyces cerevisiae NA74-3A ($\rho^-$)/ pTFE 756 have been deposited in IFO with the accession numbers IFO 10442 and IFO 10443, respectively, since July 14, 1988 and in FRI with the accession numbers FERM BP-1976 and FERM BP-1977, respectively, since July 23, 1988.


Reference Example 1


Preparation of Respiratory-Deficient Strain

Using ethidium bromide in accordance with the method described in Laboratory Course Manual for Methods in Yeast Genetics (1986) published by Cold Spring Harbor Laboratory, the respiratory-deficient strain Saccharomyces cerevisiae NA74-3A ($\rho^-$) (IFO 10431, FERM BP-1948) was isolated from Saccharomyces cerevisiae NA74-3A.

5

Reference Example 2

Preparation of Expression Vector

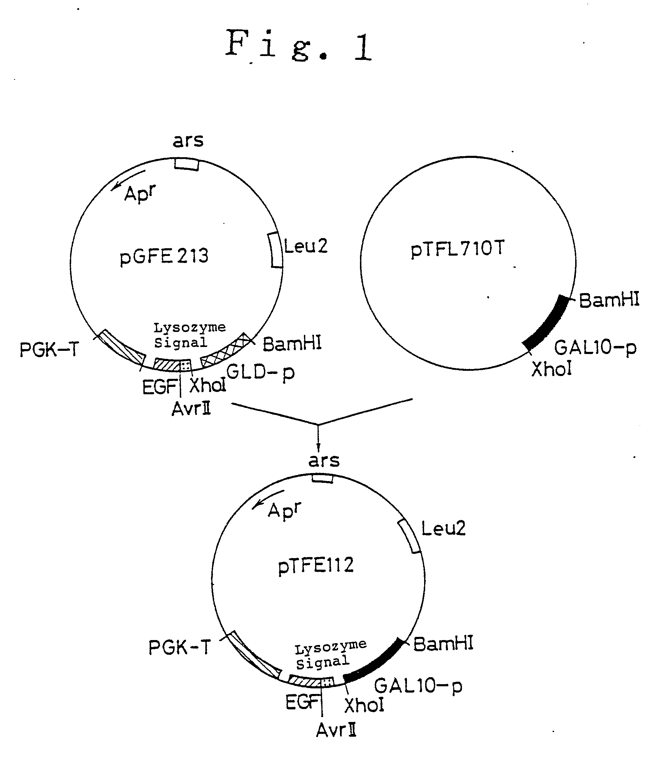

The expression vector pTFE112 was prepared by inserting a 790bp BamHI-XhoI DNA fragment [GAL10 promoter (indicated by GAL10-p in Fig. 1)] obtained from pTFL710T obtained in Reference Example 5 hereinafter described into the BamHI-XhoI site [GLD promoter site (indicated by GLD-P in Fig. 1) of the expression vector pGFE213 obtained in Reference Example 6 hereinafter described (Fig. 1).

Reference Example 3

Construction of Human Lysozyme Secretory Plasmid pGFL735

After 5 μg of Escherichia coli vector pBR322 was reacted with 1.5 units of the restriction enzyme Ball in 40 μl of a reaction solution [10 mM Tris-HCl (pH 7.5), 10 mM MgCl₂, 1mM dithiothreitol] at 37°C for 5 hours, the reaction mixture was treated with phenol and then a DNA fragment was precipitated with ethanol in accordance with known methods. To this DNA fragment, 50 ng of a phosphorylated XhoI linker d-[pCCTCGAGG] (New England Biolabs; U.S.A.) was added and they were ligated with each other by a T4 DNA ligase according to known methods.

Escherichia coli DHI was transformed with this reaction solution and a plasmid was extracted from the ampicillin-resistant and tetracycline-resistant transformant thus obtained, according to the alkaline extraction method [H. C. Birnboim and J. Doly, Nucl. Acids Res. 7, 1513 (1979)] to obtain a plasmid pBR322X having a XhoI site in place of the Ball site.

In the report of Ikehara et al. [Chem. Pharm. Bull. 34, 2202 (1986)], the human lysozyme gene is prepared, for example, from the 52 oligonucleotide blocks shown in Table 1.

Table 1

| Upper Strand No. | | Lower Strand No. | |
|---|---|---|---|
| U1 | TCGAGATGAAGGTTT | L26 | TCGAGCTATTAAAC |
| U2 | TTGAGAGATGCGAAT | L25 | ACCACAACCTTGAAC |
| U3 | TAGCCAGAACTTTGAAG | L24 | GTATTGTCTGACATC |
| U4 | AGATTGGGTATGGAC | L23 | TCTATTTTGGCATCT |
| U5 | GGCTACCGTGGTATT | L22 | GTTTCTCCAAGCGAC |
| U6 | TCTTTAGCCAACTGG | L21 | CCAGGCTCTAATACCCTG |
| U7 | ATGTGTCTTGCTAAG | L20 | TGGGTCACGGACAAC |
| U8 | TGGGAATCCGGCTATAAC | L19 | TCTCTTAGCGCAGGC |
| U9 | ACTAGAGCTACCAAT | L18 | AACAGCATCAGCAAT |
| U10 | TACAACGCTGGCGAC | L17 | GTTGTCCTGAAGC |
| U11 | CGTTCTACAGACTATGG | L16 | AAAGCTGAGCAAGAT |
| U12 | TATTTTCCAAATTAACT | L15 | AAGTGACAGGCGTTGAC |
| U13 | CTAGATATTGGTG | L14 | GGCACCTGGAGTCTTGC |
| U14 | TAACGATGGCAAGACTC | L13 | CATCGTTACACCAATAT |
| U15 | CAGGTGCCGTCAACGCC | L12 | CTAGAGTTAATTTGG |
| U16 | TGTCACTTATCTTGC | L11 | AAAATACCATAGTCTGT |
| U17 | TCAGCTTTGCTTCAG | L10 | AGAACGGTCGCCAGC |
| U18 | GACAACATTGCTGAT | L9 | GTTGTAATTGGTAGC |
| U19 | GCTGTTGCCTGCGCT | L8 | TCTAGTGTTATAGCCG |
| U20 | AAGAGAGTTGTCCGT | L7 | GATTCCCACTTAGCAAG |
| U21 | GACCCACAGGGTATT | L6 | ACACATCCAGTTGGC |
| U22 | AGAGCCTGGGTCGCT | L5 | TAAAGAAATACCACG |
| U23 | TGGAGAAACAGATGC | L4 | GTAGCCGTCCATACC |
| U24 | CAAAATAGAGATGTC | L3 | CAATCTCTTCAAAGT |
| U25 | AGACAATACGTTCAAGG | L2 | TCTGGCTAATTCGCATC |
| U26 | TTGTGGTGTTTAATAGC | L1 | TCTCAAAAACCTTCATC |

In Table 1, CGAGAGATGCGAAT was synthesized as U2-taq in place of U2, and TCTGGCTAATTCGCATCTCT was synthesized as L2-taq in place of U2, according to the report of Ikehara et al. Then, using fragments U2-taq, U3 to U26, L2-taq and L3 to L26, the respective hybrids of oligonucleotide blocks were formed according to the report of Ikehara et al. After each of these groups was ligated by the method described in Example 2 of Japanese Patent unexamined Publication No. 61-158793 (1986) which corresponds to EP-181,634, both 5´-termini were enzymatically phosphorylated to obtain a human lysozyme gene fragment.

In a separate reaction, 2.6 μg of the plasmid pBR322X was reacted with 6 units of the restriction enzyme XhoI and 6 units of the restriction enzyme ClaI in 35 μl of a reaction solution [33 mM acetate buffer (pH 7.9), 66 mM potassium acetate, 10 mM magnesium acetate, 0.5 mM dithiothreitol, 0.01 % BSA] at 37°C for 1 hour. Then, the solution was deproteinized with phenol and DNA was precipitated with cold ethanol. This DNA fragment (200 ng) was mixed with 100 ng of the human lysozyme gene fragment prepared above, and allowed to ligate with each other in 10 μl of a reaction solution [66 mM Tris-HCl (Ph 7.6), 10 mM ATP, 10 mM spermidine, 100 mM MgCl₂, 150 mM DTT, 2 mg/ml BSA, 5 units of T4 DNA ligase] at 14°C overnight. Using this reaction solution, Escherichia coli DHI was transformed according to the method of Cohen et al. [Proc. Natl. Acad. Sci. U.S.A. 69, 2110 (1972)]. A plasmid was isolated from the transformant thus obtained according to the alkaline extraction method previously mentioned. Its molecular weight and cleavage pattern by restriction enzymes were examined and pLYS221 in which the human lysozyme gene fragment was inserted was obtained. The EcoRI-XhoI fragment of pLYS221 was isolated and its nucleotide sequence was determined in accordance with the dideoxynucleotide synthetic chain termination method. As a result, the TaqI-XhoI fragment of the human lysozyme gene was obtained exactly as assumed.

This sequence codes for the Glu-4 to the Val-130 of the amino acid sequence of human lysozyme.

The Leu-4, the Ile-6 and the Val-130 of the known amino acid sequence of the egg white lysozyme signal peptide [A. Jung et al. Proc. Natl. Acad. Sci. 77, 5759 (1980)] were replaced with Phe, Leu and Ala, respectively. These substitutions in the lysozyme nucleotide sequence were chosen in consideration of the following points for higher expression.

(1) Codons which are frequently used in yeast are preferentially selected;

(2) To enhance the expression, the sequence of an yeast PGK gene is used upstream from ATG; and

(3) The construction of a hybrid signal is possible

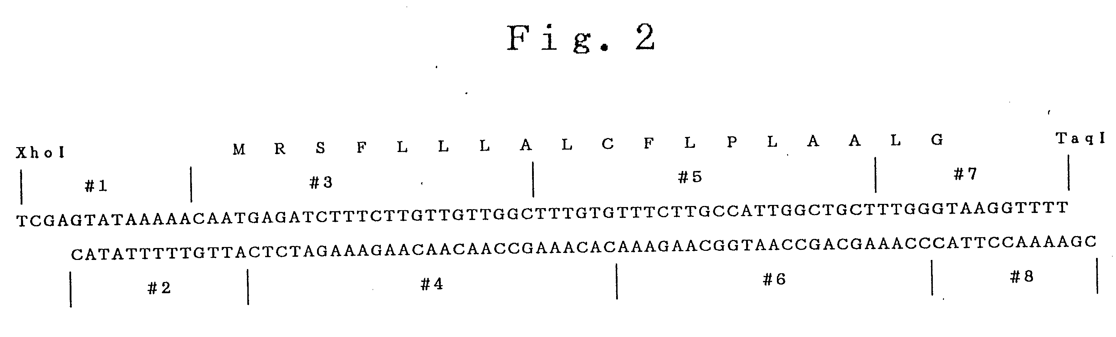

The nucleotide sequence thus synthesized is shown in Fig. 2. There are provided an XhoI site at the 5'-terminus and a TaqI site at the 3'-terminus containing the human lysozyme-encoding region. The whole sequence consists of 8 oligonucleotide blocks (#1-#8), which were prepared by the phosphoamidite method [M. H. Caruthers et al. Tetrahedron Letters 22, 1859 (1981)].

The oligonucleotide blocks #2 to #7 were first mixed with each other in 10μl (5 μg) portions, to which were further added 20μl of the kinase buffer of a 10-fold concentration (0.5 M Tris-HCl, 0.1 M MgCl₂, 0.1 M mercaptoethanol, pH 7.6), 20μl of 10 mM ATP, 20μl (50 u) of T4 polynucleotide kinase (Takara Shuzo Inc.) and 80 μl of distilled water. The mixture was then reacted at 37°C for 2 hours and thereafter treated at 65°C for 20 minutes to stop the reaction. to this reaction mixture were added the oligonucleotide blocks #1 and #8 in 10 μl (5 μg) portions. Further, 10 μl of T4 ligase (New England Biolabs, U.K.) was added thereto and the mixture was reacted at 14°C overnight. The resulting reaction mixture was subjected to 10% polyacrylamide gel electrophoresis. A 76 bp fragment was cut out and extracted from the gel by electroelution. This fragment was dissolved in 45μl of distilled water, to which were added 6 μl of the kinase buffer of a 10-fold concentration previously mentioned, 6μl of 10 mM ATP and 2 μl (5 u) of the T4 polynucleotide kinase previously mentioned. The mixture was reacted at 37°C for 1 hour and then stored at -20°C.

In Fig. 2, there is adopted a single letter expression for the amino acids (Rule Confirmed by IUPAC-IUB Biochemistry Committee on Nomenclature).

Example:

A: Alanine

B: Aspartic acid or asparagine

C: Cysteine

D: Aspartic acid

E: Glutamic acid

F: Phenylalanine

G: Glycine

H: Histidine

I: Isoleucine

K: Lysine

L: Leucine

M: Methionine

N: Asparagine

P: Proline

Q: Glutamine

R: Arginine

S: Serine

T: Threonine

V: Valine

W: Tryptophan

Y: Tyrosine

Z: Glutamic acid or glutamine

X: Unknown or other amino acids The plasmid pLYS221 (236 μg) prepared above was treated with 120 μ of EcoRI (Nippon Gene Inc.) and 120 u of XhoI (Nippon Gene Inc.) at 37°C for 2 hours to cut out a fragment of the human lysozyme-encoding region. This fragment was further treated with 26 u of TaqI (Nippon Gene Inc.) at 65°C for 1 hour to cut out a fragment of the human lysozyme-encoding region from which a part of the N-terminal portion was deleted.

About 1 μg of this fragment was mixed with 0.5 μg of a DNA fragment coding for the signal sequence described above and the mixture was reacted in the presence of 800 u of the T4 ligase previously mentioned at 16°C for 16 hours, followed by treatment with XhoI (42 u).

The XhoI fragment (10 ng) thus obtained was mixed with 1 ng of a fragment obtained by treating the yeast expression vector pGLD906-1 [Japanese Patent Unexamined Publication No. 61-43991 (1986), which corresponds to EP-171,908] with XhoI, and both were ligated with each other in the presence of T4 ligase.

Escherichia coli DHI was transformed with the resulting reaction mixture by the method described above to obtain a number of plasmids in which the signal sequence-encoding region and the human lysozyme gene were inserted downstream from the GLD promoter in the same direction as that of the promoter. One of such plasmids was named pGFL735 and used in the experiments hereinafter described.

Reference Example 4

Construction of Human Lysozyme Secretory Plasmid pPFL725T

A 0.28 kb AkhaIII-SalI fragment containing PGK terminator was isolated from the modified HBsAg P31 expression plasmid pGLD P31-Rct (European Patent Publication No. 0235430). An SalI linker pGGTCGACC was ligated with this fragment by T4 DNA ligase, followed by treatment with SalI. The 0.28 kb SalI fragment and the SalI linker not ligated with the fragment were separated by agarose gel electrophoresis and the 0.28 kb SalI fragment was obtained from agarose gel by the isolating method of DNA using DEAE cellulose paper [G. Winberg and L. Hammarskjold, Nucl. Acids Res. 8, 253 (1980)].

The human lysozyme secretory plasmid pGEL125 [K. Yoshimura et al., Biochem. Biophys. Res. Commun. 145, 712 (1987), FERM BP-1345] using the egg white lysozyme signal peptide was cleaved with BamHI and XhoI, from which a 1.5 kb BamHI-XhoI fragment containing the GLD promoter and the egg white lysozyme signal peptide - the human lysozyme-encoding region, and the residual 8.4 kb BamHI-XhoI fragment were separated by agarose gel electrophoresis. Then, each of them was isolated.

The 0.28 kb SalI fragment containing the PGK terminator was ligated by T4 DNA ligase to the 3'-terminus (XhoI site) of the above-described 1.5 kb BamHI-XhoI fragment containing the GLP promoter, the egg white lysozyme signal peptide-encoding region and the human lysozyme encoding region, and then subjected to agarose gel electrophoresis to isolate a 1.8 kb BamHI-SalI fragment.

The 1.8 kb BamHI-SalI fragment thus obtained was ligated with the 8.4 kb BamHI-XhoI fragment by T4 DNA ligase. Using the resulting fragment, Escherichia coli DHI was transformed. A plasmid was prepared from the ampicillin-resistant transformant, and was named pGEL125T.

From the human lysozyme secretory plasmid pGFL735 obtained in Reference Example 3, a 0.5 kb XhoI fragment coding for the modified signal peptide and human lysozyme was isolated. On the other hand, the expression vector pPHO17-1 (European Patent Publication No. 0235430) having the PHO-5 promoter was cleaved with XhoI and then ligated with the 0.5 kb XhoI fragment, followed by transformation of Escherichia coli. A plasmid was prepared from the ampicillin-resistant transformant thus obtained, and was named pPFL725.

Each of the plasmids pGEL125T and pPFL725 obtained as described above had XbaI cleavage sites upstream from the promoter and in the human lysozyme-encoding region. Both plasmids were cleaved with XbaI, and a 1.7 kb XbaI fragment from pPFL725 was ligated with a 7.9 kb XbaI fragment from pGEL125T by T4 DNA ligase, followed by transformation of Escherichia coli DHI. A plasmid was isolated from the ampicillin-resistant transformant thus obtained, and was named pPFL725T.

Reference Example 5

Construction of Human Lysozyme Secretory Plasmid pTFL710T

A 2.3 kb XbaI fragment and a 7.9 XbaI fragment were isolated from the human lysozyme secretory plasmid pGFL735 obtained in Reference Example 3 and from the plasmid pGEL125T obtained in Reference Example 4, respectively. Both fragments were then ligated with each other by T4 DNA ligase, followed by transformation of Escherichia coli DHI. A plasmid was isolated from the ampicillin-resistant transformant, and was named pGFL735T.

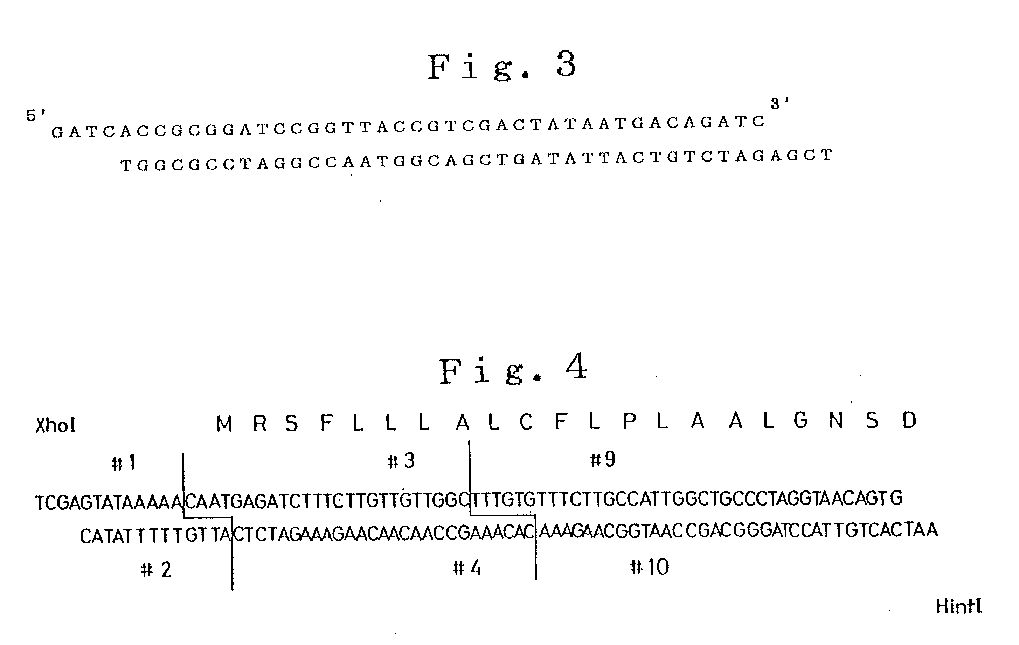

An EcoRI-XhoI adapter was ligated by T4 DNA ligase with a 0.7 kb BglII-EcoRI fragment containing the Gal 10 promoter obtained from a plasmid p286, which was obtained by replacing a 275 bp SalI-BamHI fragment located upstream from the Gal 10 promoter of the plasmid pBM150 [Mol. Cell. Biol. 4, 1440 (1984)] with a multi-linker (Fig. 3) of about 40 bp having restriction enzyme sites such as SacII, BamHI, SalI and BglII. The resulting product was then treated with BglII and XhoI, and a 0.7 kb BglII-XhoI fragment was isolated by agarose gel electrophoresis.

On the other hand, a 1.1 kb BamHI-XhoI fragment containing the GLD promoter of the plasmid pGFL735T was removed, and a BglII-XhoI fragment containing the Gal 10 promoter was inserted therein, followed by transformation of Escherichia coli DHI. A plasmid was isolated from the ampicillin-resistant transformant thus obtained, and was named pTFL710T. Saccharomyces cerevisiae NA74-3A was transformed with the plasmid pTFL710T to give a transformant Saccharomyces cerevisiae NA74-3A/pTFL710T (IFO 10432, FERM BP-2090).

Reference Example 6

Construction of Human EGF Secretory Plasmid pGFE213

The oligonucleotide block #9 and the oligonucleotide block #10 shown in Fig. 4 were chemically synthesized in place of the oligonucleotide blocks #5 and #7 and the oligonucleotide blocks #6 and #8 shown in Fig. 2 of Reference Example 3, respectively, and ligated with the oligonucleotide blocks #1 to #4 by T4 DNA ligase to prepare a 76 bp XhoI-HinfI fragment coding for a modified type of signal peptide and the N-terminal region of human epidermal growth factor (EGF). A 0.16 kb HinfI-PstI fragment coding for human EGF from which a portion of the N-terminus was removed was isolated from the plasmid pTB370 [Taniyama et al., J. Takeda Res. Lab. 45, 136 (1986), Japanese Patent Unexamined Publication No. 61-88881 (1986), which corresponds to EP-177915], and to the 5′-terminus thereof was ligated the 76 bp XhoI-HinfI fragment described above by T4 DNA ligase, followed by treatment with XhoI and PstI to isolate a 0.24 kb XhoI-PstI fragment. A PstI-SmaI adapter was ligated with this fragment by T4 DNA ligase, followed by treatment with XhoI and SmaI to isolate a 0.24 kb XhoI-SmaI fragment coding for the modified type of signal peptide and human EGF.

There was cleaved with XhoI and SmaI a plasmid pGFL735Sm obtained by modifying the XhoI site located downstream from the human lysozyme-encoding region of the plasmid pGFL735 obtained in Reference Example 3 to an SmaI site, and a DNA fragment coding for the modified type of signal peptide and human lysozyme was removed. In place of the removed DNA fragment the 0.24 kb XhoI-SmaI fragment described above was inserted to obtain a plasmid pGFE101.

A 0.28 kb AhaIII-XhoI fragment containing the PGK terminator was isolated fromt he plasmid pGLD P31-RcT previously mentioned, and inserted in the SmaI-SalI site of the plasmid pSP64 (Riboprobe Inc. U.S.A.) to obtain a plasmid pSP64-T (PGK). An EcoRI-PstI fragment containing the PGK terminator was isolated from this plasmid, and the PstI-SmaI adapter and a SmaI-EcoRI adapter were added thereto, followed by insertion of the resulting product into the SmaI site of the plasmid pGFE101 described above to obtain a human EGF secretory plasmid pGFE213.

Example 1

Preparation of Rat Kinase C α Gene

A 3.3 kbp DNA fragment having the PKC α gene region was prepared fromt he expression vector pTB755 for animal cells (European Patent Publication no. 251244, Example 5) coding for a rat protein C kinase α cDNA by complete digestion with EcoRI. This fragment was inserted into PUC18 (Takara Shuzo Inc., Japan) digested with EcoRI. Then, the recombinant DNA fragment thus obtained was digested with NcoI and PstI to prepare a 140 bp NcoI-PstI fragment. This recombinant DNA fragment was also digested with PstI and StuI to prepare a 2.0 kbp PstI-StuI fragment.

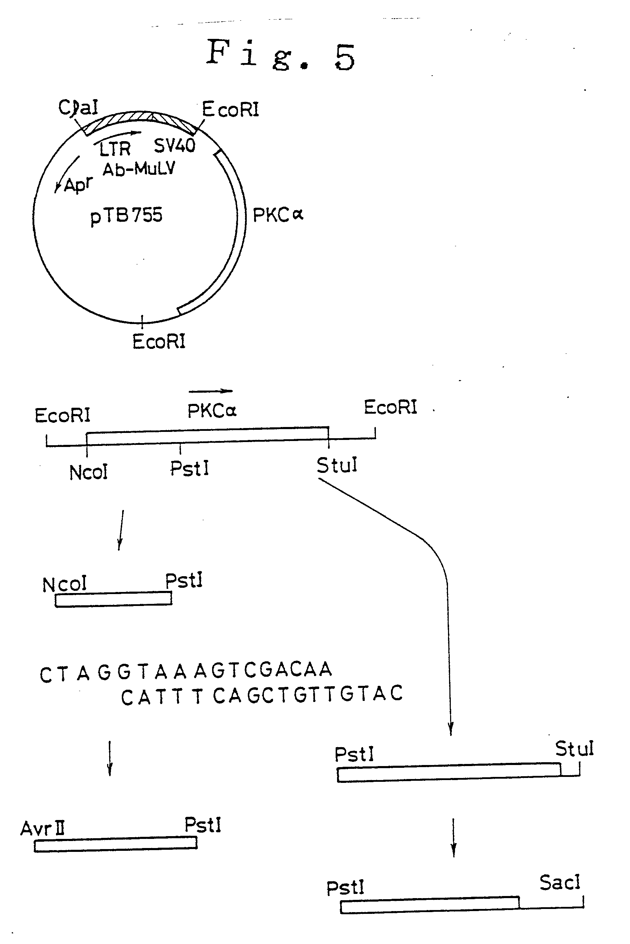

In the former, an adapter shown in Fig. 5 was ligated to the NcoI site by T4 ligase to obtain a 150 bp AvrII-PstI DNA fragment.

In the latter, the SacI linker of the 8-mer pCGAGCTCG (New England Biolabs, U.S.A.) was ligated to the StuI site by T4 ligase, followed by digestion with SacI to obtain a 2.0 kbp PstI-SacI fragment (Fig. 5).

Example 2

Construction of Expression Plasmid (1)

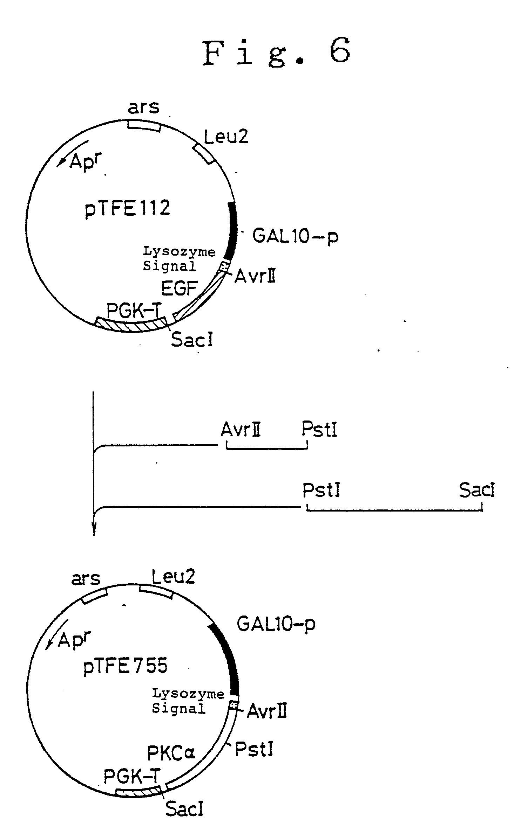

The expression plasmid pTFE112 for yeast obtained in Reference Example 2 was digested with restriction enzymes AvrII and SacI to obtain a 9.6 kbp DNA fragment. Into this 9.6 kbp fragment were inserted the 150 bp fragment and the 2.0 kbp fragment obtained in Example 1, thereby an expression plasmid pTFE755 was prepared(Fig. 6).

Example 3

Construction of Expression Plasmid (2)

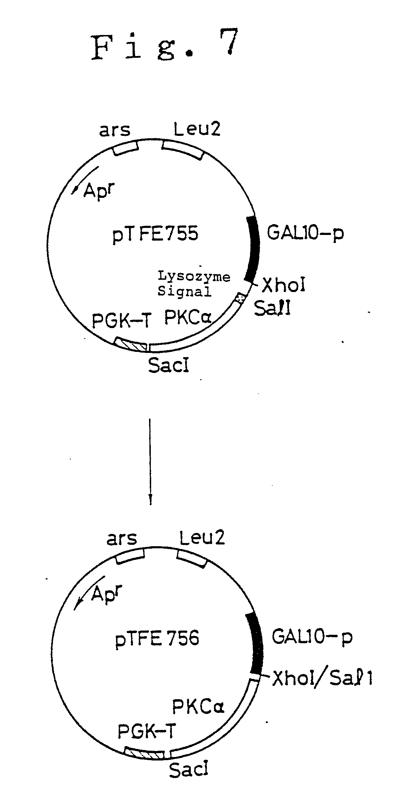

The plasmid pTFE755 obtained in Example 2 was digested with SalI and SacI to provide a 2.2 kbp fragment, and the fragment was inserted in the XhoI-SacI site of pTFE112, thereby an expression plasmid pTFE756 was prepared(Fig. 7).

Example 4

Preparation of Yeast Transformant, Cultivation Thereof and Expression of PKC α Protein

The yeast Saccharomyces cerevisiae NA74-3A($\rho^-$) described in Reference Example 1 was transformed with the plasmids pTFE755 or pTFE756 obtained in Examples 2 or 3 to give transformants Saccharomyces cerevisiae NA74-3A($\rho^-$)pTFE755 IFO 10442, FERM BP-1976). and Saccharomyces cerevisiae NA74-3A-($\rho^-$)/pTFE756, IFO 10443, FERM BP-1977), respectively.

Each of these yeast transformants was then cultivated with shaking at 30°C for 3 days in 5 ml of a medium (containing 3 g of $K_2HPO_4$, 50 g of glucose, 4 g of asparagine, 100mg of L-histidine, 0.1 mg of KI, 500 mg of $MgSO_4.7H_2O$, 330 mg of $CaCl_2.2H_2O$, 0.4 mg of $CuSO_4.5H_2O$, 2.5 mg of $FeSO_4.7H_2O$, 0.4 mg of $MnSO_4.4H_2O$, 0.2 mg of $(NH_4)_3PO_4.12MoO_3.3H_2O$, 3.1 mg of $ZnSO_4.7H_2O$, 10 mg of inositol, 0.2 mg of thiamine, 0.2 mg of pyridoxine, 0.2 mg of Ca-pantothenate, 0.2 mg of niacin and 0.002 mg of biotin, per liter). Then, 0.5 ml of the culture was transferred to 4.5 ml of another medium having the same composition as above, and cultivated at 30°C for 1 day. Further, 2 ml of the culture thus obtained was transferred to 18 ml of a fresh medium (containing 400 mg of $K_2HPO_4$, 80 g of glucose, 5 g of asparagine, 300 mg of L-histidine, 2.0 g of KCl, 0.1 mg of KI, 650 mg of $MgSO_4.7H_2O$, 429 mg of $CaCl_2.2H_2O$, 10 g of galactose, 25 mM of Tris-maleic acid (pH 6.5), 0.4 mg of $CuSO_4.5H_2O$, 2.5 mg of $FeSO_4.7H_2O$, 0.4 mg of $MnSO_4.4H_2O$, 0.2 mg of $(NH_4)_3PO_4.12MoO_3.3H_2O$, 3.1 mg of $ZnSO_4.7H_2O$, 10 mg of inositol, 0.2 mg of thiamine, 0.2 mg of pyridoxine, 4.0 mg of Ca-pantothenate, 4.0 mg of niacin and 0.040 mg of biotin, per liter), and cultivated with shaking at 30°C for 3 days. After 72 hours, the culture was sampled and subjected to centrifugation at 10,000 X g for 10 minutes to separate cells from a supernatant.

The above-mentioned cells (200 mg in wet cell weight) were suspended in 500 μl of an extraction buffer [0.1 % Tween 20, 7.5 M urea, 15 mM EDTA, 2 mM PMSF (phenylmethylsulfonyl fluoride), 0.2 mM APMSF (p-amidinophenylmethane-sulfonyl fluoride hydrochloride), 5 mM EGTA (ethyleneglycol-bis-(β-aminoethyl ether)N, N, N´, N´-tetraacetic acid), 0.1 M phosphate buffer, pH 7.2], and 1 g of glass beads was added thereto. The cells were thereafter disrupted by using a Vortex mixer (Scientific Industries, Inc,. U.S.A.) at 4°C for 30 minutes, and then a supernatant was collected by centrifugation at 12,000 rpm for 10 minutes. To this supernatant was added the same amount of SDS-gel sample buffer [V. K. Laemmli et al., Nature 227, 680 (1970)] and heated at 100°C for 10 minutes to solubilize proteins. Then, the solubilized proteins were fractionated by 10 % SDS-polyacrylamide electrophoresis, and subsequently transferred to a nitrocellulose filter by the known western blotting method [H. Towbin et al., Proc. Natl. Acad. Sci. U.S.A. 76, 4350 (1979)]. Screening for a PKC α protein was conducted by using the known anti-PKCα monoclonal antibody (M. C. Clone et al., Amersham Inc.). As a result, it was confirmed that the PKC α protein having a nearly identical molecular weight was expressed.

With respect to the bands obtained by the above-mentioned western blotting method, the concentration of protein kinase C α protein was determined by using a densitometer. In the case of Saccharomyces cerevisiae NA74-3A($\rho^-$)/pTFE755, the determined value was about 36 μg/ml of medium. Similarly, in the case of Saccharomyces cerevisiae NA74-3A($\rho^-$)/pTFE756, the determined value was about 3 μg/ml of

medium. These results show that the protein was produced in remarkable amounts by means of the transforming yeasts.

Example 5

Determination of PKC α Activity

The cells (200 mg in wet cell weight) obtained by cultivation according to the method of Example 4 were suspended in 500 μl of 0.1 M Tris-HCl buffer (pH 7.5) containing 20 % glycerol, 1 mM APMSF and 5 mM EGTA, and 1 g of glass beads was added thereto. The cells were thereafter disrupted, and then a supernatant was collected by centrifugation at 12,000 rpm for 10 minutes. The activity of protein kinase C was determined by using this supernatant according to the method of Kikkawa et al. [U. Kikkawa et al., J. Biol. Chem. 257, 13341 (1982)]. As a result, it was confirmed that the supernatant had phosphorylated histone type I and the activity was dependant on phorbol esters, phospholipids and $Ca^{++}$. For control examples in which the PKC α protein was not expressed, the protein kinase activity was not observed.

**Claims**

1. An expression vector for a protein kinase C protein, which contains a DNA that encodes said protein, and which is capable of autonomous replication in yeasts.

2. The vector according to claim 1, comprising pTFE 755.

3. The vector according to claim 1, comprising pTFE 756.

4. A yeast transformed with an expression vector for a protein kinase C protein which vector contains a DNA encoding said protein and is capable of autonomous replication in yeasts.

5. The yeast according to claim 4, which comprises a respiratory deficient strain.

6. The yeast according to claim 5, which comprises a Saccharomyces cerevisiae.

7. The transformant according to claim 6, which comprises Saccharomyces cerevisiae NA74-3A ($\rho^-$)-/pTFE 755.

8. The transformant according to claim 6, which comprises Saccharomyces cerevisiae NA74-3A ($\rho^-$)-/pTFE 756.

9. A method of producing a protein kinase C protein, which comprises: cultivating in a culture medium a yeast transformed with an expression vector for a protein kinase C protein, which vector contains a DNA that encodes the protein, and which is capable of autonomous replication in yeasts; accumulating said protein in the culture medium; and harvesting said protein.

10. A method according to claim 9 wherein the yeast is a respiratory deficient strain.

11. A method according to claim 9 wherein the yeast is Saccharomyces cerevisiae.

12. A method according to claim 9 wherein the yeast is Saccharomyces cerevisiae NA74-3A ($\rho^-$)/pTFE 755.

13. A method according to claim 9 wherein the yeast is Saccharomyces cerevisiae NA74-3A ($\rho^-$)/pTFE 756.

# Fig. 1

# Fig. 2

```
Xho I                   M  R  S  F  L  L  L  A  L  C  F  L  P  L  A  A  L  G              Taq I

      #1            |            #3              .            |              #5              |        #7        |
 |                                                                                                             |
TCGAGTATAAAAACAATGAGATCTTTCTTGTTGTTGGCTTTGTGTTTCTTGCCATTGGCTGCTTTGGGTAAGGTTTT
          CATATTTTTGTTACTCTAGAAAGAACAACAACCGAAACACAAAGAACGGTAACCGACGAAACCCATTCCAAAAGC
 |        #2        |              #4              |              #6              |        #8        |
```

# Fig. 3

5' GATCACCGCGGATCCGGTTACCGTCGACTATAATGACAGATC 3'

TGGCGCCTAGGCCAATGGCAGCTGATATTACTGTCTAGAGCT

EP 0 358 325 A1

# Fig. 4

XhoI

M R S F L L L A L C F L P L A A L G N S D

#1                    #3              #9

TCGAGTATAAAAACAATGAGATCTTTCTTGTTGTTGGCTTTGTGTTTCTTGCCATTGGCTGCCCTAGGTAACAGTG

CATATTTTTGTTACTCTAGAAAGAACAACAACCGAAACACAAAGAACGGTAACCGACGGGATCCATTGTCACTAA

#2                    #4              #10

HinfI

# Fig. 5

CTAGGTAAAGTCGACAA
CATTTCAGCTGTTGTAC

# Fig. 6

# Fig. 7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 801 303 (GENETICS INSTITUTE INC.) <br> * Claims; page 12, line 35 - page 13, line 4 * | 1-4,9-11 | C 12 N 15/81 <br> C 12 N 15/54 <br> C 12 N 9/12 <br> C 12 N 1/18 // <br> (C 12 N 1/18 <br> C 12 R 1:865) |
| X | EP-A-0 251 244 (TAKEDA CHEMICAL INDUSTRIES LTD) <br> * Claims; page 6, line 4 * | 1-4,9-11 | |
| P,X | EP-A-0 317 209 (TAKEDA CHEMICAL INDUSTRIES) <br> * Caims 1,5 * | 5-8,10,12,13 | |
| A | TRENDS IN BIOTECHNOLOGY, vol. 5, no. 2, February 1987, pages 53-56, Elsevier Science Publishers B.V., Amsterdam, NL; S.M. KINGSMAN et al.: "The production of mammalian proteins in Saccharomyces cerevisiae" | | |

TECHNICAL FIELDS
SEARCHED (Int. Cl.5)

C 12 N

The present search report has been drawn up for all claims

| Place of search | Date of completion of the search | Examiner |
|---|---|---|
| THE HAGUE | 16-10-1989 | VAN PUTTEN A.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)